Europäisches Patentamt

European Patent Office

Office européen des brevets

(11) Veröffentlichungsnummer: **0 300 163 B1**

# EUROPÄISCHE PATENTSCHRIFT

(45) Veröffentlichungstag der Patentschrift: **22.07.92**

(21) Anmeldenummer: **88108298.6**

(22) Anmeldetag: **25.05.88**

(51) Int. Cl.⁵: **C07D 301/14**

(54) Verfahren zur Herstellung von Epoxiden.

(30) Priorität: **18.07.87 DE 3723843**

(43) Veröffentlichungstag der Anmeldung:
**25.01.89 Patentblatt 89/04**

(45) Bekanntmachung des Hinweises auf die
Patenterteilung:
**22.07.92 Patentblatt 92/30**

(84) Benannte Vertragsstaaten:
**AT BE CH DE FR GB IT LI NL**

(56) Entgegenhaltungen:
**EP-A- 0 055 387**
**DE-B- 1 015 782**
**GB-A- 964 722**
**US-A- 2 838 524**

(73) Patentinhaber: **Degussa Aktiengesellschaft**
**Weissfrauenstrasse 9**
**W-6000 Frankfurt am Main 1(DE)**

(72) Erfinder: **Siegmeier, Rainer, Dr.**
**Anspacher Strasse 35**
**W-6380 Bad Homburg(DE)**
Erfinder: **Wirthwein, Rolf, Dr.**
**Auguste-Viktoria-Strasse 45**
**W-5040 Brühl(DE)**
Erfinder: **Brandt, Udo**
**Fürstenbergstrasse 4**
**W-6450 Hanau 9(DE)**

**Beschreibung**

Eine der bekanntesten Reaktionen zur Herstellung von Epoxiden ist die von Prileschajew, nämlich die Umsetzung von Olefinen mit Percarbonsäuren, siehe Ber. dtsch. chem. Ges. 42, 4811, (1909).

Bei dieser Reaktion werden Percarbonsäuren benutzt, die durch einfache Umsetzung von wäßrigem Wasserstoffperoxid mit Carbonsäuren oder deren Derivaten in Gegenwart von Protonen-Donatoren, wie z.B. starken Mineralsäuren, gewonnen werden können, und zwar in einer Gleichgewichtsreaktion, siehe Methodicum Chimicum, Band 5, Seite 737, 1978.

Bei der Übertragung dieser so einfach erscheinenden Prileschajew-Reaktion in die Technik zeigten sich jedoch Schwierigkeiten, die zum Teil auf die gewisse Reaktionsträgheit der Doppelbindungen in $\alpha$-Olefinen, zum anderen auf die Bedingungen bei der Umsetzung, wie die Anwesenheit von Wasser bzw. von starken Mineralsäuren, zurückzuführen waren. Letztere wirken bekanntlich auf die schon gebildeten Oxiranringe durch Ringöffnung ein.

Aber auch die Verwendung möglichst wasserfreier Systeme wie in dem Verfahren der DE-AS 1 230 005 brachte keine Verbesserung in Bezug auf Verminderung der Nebenproduktbildung und Ausbeuteerhöhung.

In der Technik werden häufig sogenannte Gleichgewichtspercarbonsäuren eingesetzt. Sie entstehen durch direkte säurekatalysierte Reaktion zwischen Carbonsäuren und Wasserstoffperoxid. Als saurer Katalysator werden starke Säuren, wie Mineralsäuren, vor allem Schwefelsäure, verwendet. Die mit den niederen aliphatischen Carbonsäuren erhaltenen Lösungen von Peroxysäuren, z.B. von Peressigsäure, enthalten neben der Persäure noch die eingesetzte Carbonsäure sowie restliches Wasserstoffperoxid und Wasser. Allerdings werden unter " Gleichgewichtspercarbonsäure" nicht nur die durch wirkliche Gleichgewichtseinstellung entstandenen Gemische verstanden, sondern auch Mischungen, die neben der gebildeten Persäure noch die Carbonsäure, katalytische Mengen der starken Säure, wie auch bevorzugt Wasserstoffperoxid und Wasser enthalten (EP-PS 0 055 387).

So besteht z.B. die handelsübliche "Gleichgewichtsperessigsäure" aus etwa 40 Gew.-% Peressigsäure, 40 Gew.-% Essigsäure, 5 Gew.-% Wasserstoffperoxid, 14 Gew.-% Wasser und 1 Gew.-% Schwefelsäure (Ullmanns Enzyklopädie der technischen Chemie, 4. Aufl., Band 17, 669).

Da aber bei Verwendung der Gleichgewichtspercarbonsäure Nebenreaktionen vor allem durch die Anwesenheit von Säure eintraten, versuchte man, den Einfluß der in der Gleichgewichtspercarbonsäure vorhandenen starken Mineralsäure zu verringern, z.B. durch Neutralisation der Schwefelsäure mit Puffersalzen, bevor die in diesem Fall eingesetzte Gleichgewichtsperessigsäure mit dem Olefin zusammengebracht wurde, siehe Swern, Organic Peroxides, Vol II, Wiley-Interscience, 1971, Seite 436. Diese Maßnahme soll jedoch bei säurelabilen Epoxiden nicht ausreichen; daher wird nach "Swern" loc. cit., Seite 399 und 436, dem Reaktionsgemisch eine solche Menge an schwachen Basen zugegeben, daß nicht nur die Schwefelsäure, sondern auch zum Teil die gebildete Essigsäure neutralisiert wird.

Nach dem Verfahren der DE-OS 28 35 940 wird die Epoxydierung von ungesättigten Terpenkohlenwasserstoffen mit Gleichgewichtsperessigsäure, die also bestimmte Mengen an freiem Wasserstoffperoxid und Essigsäure, sowie Schwefelsäure und Wasser, enthält, in Anwesenheit von organischen Lösungsmitteln durchgeführt und die freie Säure mit festen, säurebindenden Mitteln neutralisiert.

Schließlich werden in der EP-PS 0 055 387 zur Säurebindung der freien Säuren in der Gleichgewichtsperessigsäure als auch während der Epoxydierungsreaktion selbst sowohl der Gleichgewichtsperessigsäure als auch dem Reaktionsgemisch säurebindende Mittel zugesetzt. Auch hier wird in Anwesenheit organischer Lösungsmittel gearbeitet.

In keinem der bisher bekannten Verfahren wird jedoch ein bestimmter pH-Wert angegeben, bei dem die Epoxydierung durchgeführt werden soll; man begnügte sich mit Verhältnisangaben zwischen dem säurebindenden Mittel und dem zu bindenden Säuregehalt.

Ferner wird bisher die Reaktion in Gegenwart von Feststoffen durchgeführt. Dies führt sowohl zu zum Teil erheblichen Rührproblemen als auch in der Regel zu längeren Reaktionszeiten. Dies trifft besonders für das Verfahren der EP-PS 0 055 387 zu.

Um die Rührfähigkeit zu verbessern, wurden entweder die Olefine im Überschuß oder organische Lösungsmittel zugesetzt, siehe EP-PS 0 055 387.

Sowohl nach der EP-PS 0 055 387 als auch der DE-OS 28 35 940 entsteht der Eindruck, größere Mengen Wasser nach Möglichkeit im Reaktionssystem zu vermeiden; nach der DE-OS 28 35 940 werden nicht nur feste, säurebindende Mittel, sondern bevorzugt sogar wasserfreie, eingesetzt.

Als säurebindende Mittel werden in erster Linie Alkalicarbonate oder -hydrogencarbonate bzw. Alkali- oder Erdalkalisalze der betreffenden umzusetzenden Carbonsäure, wie z.B. Acetate, verwendet (DE-OS 28 35 940 und EP-PS 0 055 387). Besonders bei der Bindung der Carbonsäure wurden die Carbonate oder Hydrogencarbonate bevorzugt eingesetzt, um die gebildeten Epoxide nicht zu schädigen (EP-PS 0 055 387). Stark alkalisch wirkende Stoffe, wie Alkalihydroxide, sind auch nach Ansicht der DE-OS 28 35 940 "nicht ausreichend befriedigend".

Die in den letztgenannten beiden Patentschritten beschriebenen Verfahren betreffen mehrphasige Systeme mit hohen Feststoffgehalten. In diesen Systemen wird die Reaktion unter Einsatz von überschüssiger Percarbonsäure durchgeführt, siehe die Beispiele. Unter diesen Bedingungen treten aber bei fehlender pH-Wert-Kontrolle erhebliche Zersetzungen der Percarbonsäure sowie der bevorzugt eingesetzten Carbonate und Hydrogencarbonate auf, die dann zu starker Abgasbildung führen. Diese Abgase sind nicht nur durch das Mitreißen von Lösungsmitteldampfen und eingesetztem Olefin giftig, sondern sie reichern sich auch mit dem durch Zersetzung der Persäure entstandenen Sauerstoff an und werden dadurch eventuell explosibel.

Im Epoxidationsverfahren der US 2,838,524 werden Olefine mit einer Alkanperoxosäure in Gegenwart einer ausreichenden Menge eines Säureakzeptors umgesetzt. Peroxosäure und Säureakzeptor können zwar gleichzeitig zum Olefin enthaltenden Reaktionsgemisch gegeben werden, bevorzugt wird aber die gesamte Menge Säureakzeptor vorgelegt. Eine Konstanthaltung des pH-Werts innerhalb enger Grenzen wird nicht gelehrt.

Die DE-PS 10 15 782 lehrt ein Verfahren zur Herstellung von Epoxiden aus Olefinen und Persäuren, wobei dem Reaktionsgemisch Puffersalze zugesetzt oder in diesem gebildet werden. Die Menge Puffersalze richtet sich nach dem pH-Wert, der bevorzugt zwischen 4 und 6,5 liegt. Ausweislich der Beispiele wird die Gesamtmenge an Base in fester Form vor der Umsetzung dem Reaktionsgemisch zugesetzt, Ein Konstanthalten des pH-Werts während der gesamten Epoxidation läßt sich diesem Dokument nicht entnehmen,

Im Epoxidationsverfahren der GB-A 0964722 werden Olefine mit vorgebildeter Gleichgewichtspersäure in Gegenwart des Säureanhydrids und einer ausreichenden Menge einer Base bei pH 2,5 bis 6,5 umgesetzt. Der Säureakzeptor wird in der ersten Stufe in fester Form vorgelegt, der pH-Wert wird also nicht innerhalb eines engen Bereichs konstant gehalten, sondern erst am Ende der zweiten Stufe festgestellt.

Aufgabe der Erfindung ist es nun, die Herstellung von Epoxiden unter Verwendung von Percarbonsäuren, bevorzugt von sogenannten Gleichgewichtspercarbonsäuren in Gegenwart säurebindender Mittel, unter Vermeidung von Rühr- und Abgasproblemen mit sehr guten Ausbeuten und guter Selektivität durchzuführen.

Gefunden wurde ein Verfahren zur Herstellung von Epoxiden durch Umsetzung von Olefinen mit Percarbonsäuren in Gegenwart säurebindender Mittel das dadurch gekennzeichnet ist, daß man die Epoxidationsreaktion in einem feststofffreien oder nahezu von Feststoffen freien wäßrig-organischen Zweiphasensystem bei einem konstanten pH-Wert von 3 bis 6 durchführt, wobei das Verhältnis von eingesetztem Olefin zu dem Gesamtwasser aus zugefügtem Wasser, Wasseranteil der Peressigsäure und der wäßrigen Lösung des Säurebinders auf 1 zu 0,5 bis 1 zu 5 einstellt und der vom jeweiligen Olefin abhängige, in einem Vorversuch festgestellte optimale pH-Wert während der gesamten Reaktionsdauer durch Zugabe von alkalisch wirkenden Stoffen gehalten wird.

Unter "Gleichgewichtsperessigsäure" wird - wie oben ausgeführt wurde - nicht nur die durch Einstellung eines echten Gleichgewichtes zwischen Peressigsäure, Essigsäure, Wasserstoffperoxid und Wasser in Anwesenheit einer starken Säure erhaltene Mischung verstanden, sondern alle Mischungen aus Peressigsäure und Essigsäure, die noch Wasser, Wasserstoffperoxid und die starke Säure enthalten, siehe EP-PS 0 055 387 und DE-OS 28 35 940.

Im Unterschied zu den mit Feststoffen arbeitenden Systemen der EP-PS 0 055 387 und der DE-OS 28 35 940 wird die Reaktion zwischen dem jeweiligen Olefin und Peressigsäure in einem flüssigen bzw. nahezu von Feststoffen freien System durchgeführt. Dieses System besteht sowohl aus dem Olefin und ggf. einem organischen Lösungsmittel als auch aus einem wäßrigen Anteil, in das hinein unter Rühren die Peressigsäure eingeführt wird.

Der pH-Wert bzw. der pH-Wertbereich wird vor Beginn der Reaktion festgelegt; die optimalen Werte richten sich nach dem jeweiligen Olefin; z.B. liegt dieser Wert bei pH = 5,0 beim Einsetzen von Cyclohexen; bei pH = 6,0 beim verwenden von $\alpha$-Pinen. Durch einen optimierenden Vorversuch lassen sich die entsprechenden pH-Werte leicht feststellen.

Die Einstellung des gewünschten pH-Wertes oder pH-Wertbereiches wird bevorzugt durch wäßrige Alkalilauge, z.B. Natronlauge, vorgenommen, die in üblicher Konzentration, z.B. 30 - 50 gew.-%ig, eingesetzt wird. Das dann gebildete Alkaliacetat liegt bevorzugt nicht als Feststoff, sondern durch die gleichzeitige Anwesenheit von Wasser in gelöster Form vor und wirkt dadurch gleichzeitig als Puffermedium.

Außer Alkalihydroxiden können natürlich auch die anderen, nach dem Stand der Technik bekannten säurebindenden Mittel eingesetzt werden; auch hier bevorzugt als wäßrige Lösungen.

So kommen neben Alkalicarbonaten oder -hydrogencarbonaten bzw. den entsprechenden Erdalkaliverbindungen auch Alkali-oder Erdalkalisalze der Essigsäure infrage, ebenso Alkali-oder Erdalkalioxide.

Wesentlich für die Durchführung des erfindungsgemäßen Verfahrens ist es, daß das System möglichst frei von Feststoffen ist. Daher können die obengenannten, an sich festen Säurebinder nach dem Stand der Technik entweder gelöst oder nur in solchen Mengen eingesetzt werden, daß das im System vorhandene Wasser sie während der Reaktion bevorzugt lösen kann.

Die Menge des anwesenden Wassers ist gegenüber den auf möglichste Wasserfreiheit hinzielenden Systemen des Standes der Technik wesentlich größer. Im Gegensatz zu diesen Systemen ist die Anwesenheit des Wassers gerade erwünscht, um das Auftreten von Feststoffen und die dadurch hervorgerufenen Probleme beim Rühren auszuschließen oder stark zu vermindern.

Die Reaktion läßt sich zwar ohne organisches Lösungsmittel durchführen. Bevorzugt arbeitet man aber in Gegenwart der nach dem Stand der Technik üblichen Lösungsmittel und setzt das Olefin zu dem Lösungsmittel in einem Verhältnis von 1 : 0,5 bis 1 : 10 ein.

Das Verhältnis von eingesetztem Olefin zu Wasser, d.h. dem Gesamtwasser aus zugefügtem Wasser, dem Wasseranteil der Peressigsäure und dem bevorzugt gelöst einzusetzenden Säurebinder, soll bei 1 : 0,5 bis 1 : 5 liegen, wenn eine homogene flüssige Mischung während der Reaktion vorliegt. Dieses Verhältnis hängt vom pH-Wert ab, der umso mehr Wasser benötigt, je höher er liegt; jedoch wird man die Wassermenge so gering wie möglich halten, um die Raum-Zeitausbeute nicht unnötig zu verringern. Die pH-Wert-Messung kann durch Probennahme während der Reaktion auf analytischem Wege als auch kontinuierlich durch eine im Reaktionsgefäß angebrachte übliche pH-Elektrode durchgeführt werden. Es ist die Gegenwart der wäßrigen Phase, die erst diese Art der pH-Messung ermöglicht.

Die Reaktion wird bei Temperaturen von 0 bis 70 °C, bevorzugt bei 20 bis 50 °C, durchgeführt. Da die Epoxydationsreaktion exotherm ist, wird die Reaktionstemperatur für die Dauer der Umsetzung durch Kühlung gehalten.

Als Olefine kommen infrage:
Aliphatische endständige Mono- und Diolefine der Kettenlänge zwischen $C_4$ und $C_{30}$, außerdem verzweigte Mono- und Diolefine, wie z.B. 2-Methyl-2-buten (Isoamylen), Menthylpentadien; ferner cyclische Mono-und Diolefine, wie z.B. Cyclohexen, Cyclooctadien. Auch ungesättigte Ester, wie z.B. 3-Cyclohexenylmethyl-3-Cyclohexenylcarboxylat; auch Terpene, wie z.B. Limonen, $\alpha$-bzw. $\beta$-pinen, sowie Polyolefine, wie z.B. Polybutadien.

Ebenso sind aliphatische $\alpha$ , $\omega$-Diolefine, wie Hexadien, Octadien, Tetradecadien, Decadien einsetzbar, ferner ungesättigte Säuren,wie Linolsäure oder Linolensäure.

Das Verhältnis von Olefin zu Gleichgewichtsperessigsäure liegt bevorzugt bei 1 : 1; jedoch sind Überschüsse an Olefin bzw. Peressigsäure möglich.

Wie schon ausgeführt, ist das feststofffreie, homogene zweiphasige System bei der Durchführung der Epoxydationsreaktion bevorzugt.

Ein geringfügiger Feststoffgehalt - wie er z.B. durch ausfallende Alkali- bzw. Erdalkaliacetate hervorgerufen werden kann, ist jedoch nicht störend. Er beeinträchtigt weder die Dauer der Epoxydation noch die Rührfähigkeit des Systems.

Das erhaltene Reaktionsgemisch läßt man noch - wie üblich - eine gewisse Zeit nachreagieren, je nach Art des Olefins bei der gleichen Temperatur wie bei der Reaktion selbst oder einer etwas höheren Temperatur. Dies läßt sich durch einen Vorversuch leicht feststellen.

Anschließend werden die organische und die wäßrige Phase getrennt, die organische Phase wird ein- oder mehrmals gewaschen und gegebenenfalls bei der vorletzten Wäsche der pH-Wert der Waschlösung auf 7 gehalten. Durch Abdestillieren wird das Epoxid aus der organischen Phase isoliert.

Das erfindungsgemäße Verfahren hat gegenüber den Verfahren des Standes der Technik den großen Vorteil, durch Einhalten eines bestimmten pH-Wertes, d h. durch pH-Wert-Kontrolle, eine wesentliche Zersetzung der Gleichgewichtsperessigsäure und damit das Entstehen großer Mengen an Abgas zu vermeiden. Gleichzeitig werden aber sehr gute Ausbeuten und Selektivitäten erhalten, die besten Werte - wie gesagt - bei einem bestimmten pH-Wert innerhalb des Gebietes von pH = 3 - 6, siehe z.B. Tabelle 1.- (Es handelt sich allerdings um mehr qualitative Vergleichsversuche, die nur die Abhängigkeit der Ausbeuten von dem pH-Wert zeigen sollten).

Tabelle 1

| Olefin | pH-Wert | Epoxid-Ausbeute % der Theorie |
|---|---|---|
| Cyclohexen | 3,5 | 82,2 |
| Cyclohexen | 4,0 | 86,9 |
| Cyclohexen | 5,0 | 97,0 |
| Cyclohexen | 6,0 | 91,5 |

Ferner war nicht vorherzusehen, daß nach dem erfindungsgemäßen Verfahren trotz Anwesenheit merklicher Mengen Wasser in einem nicht neutralen, sondern sauren Milieu so gut wie keine Hydrolyse auftrat. Das Verfahren ist daher sowohl für säureempfindliche wie säureunempfindliche Epoxide einsetzbar.

Das erfindungsgemäße Verfahren wird in den nachfolgenden Beispielen näher erläutert.

Beispiel 1

In einem 1 Liter Reaktionskolben mit Rührer, Thermometer, pH-Elektrode, Rückflußkühler und 2 Zulauftrichter werden 1,0 Mol = 128,4 g (+)-$\alpha$-Pinen, 96 %ig, 321,0 g Chloroform und 192,6 g Wasser vorgelegt. Anschließend gibt man innerhalb von 30 Minuten 1,0 Mol = 190,1 g Gleichgewichtsperessigsäure, 40 %ig, hinzu, während gleichzeitig der pH-Wert der Reaktionslösung durch Zugabe von ca. 180 g 50 %iger Natronlauge bei pH = 6,0 gehalten wird. Die Reaktionstemperatur wird während dieser Zeit durch Kühlen auf 20 °C gehalten.

Man läßt noch 1 Stunde bei 2o °C unter Rühren reagieren und trennt die Phasen. Die organische Phase wird dreimal mit je 100 g Wasser gewaschen, wobei bei der zweiten Wäsche mittels Natronlauge ein pH-Wert von 7,0 eingestellt wird.

Die gewaschene organische Phase wird destillativ aufgearbeitet. Hierbei werden bei einem Olefin-Umsatz von 83 % vom Einsatz 120,5 g = 0,792 Mol (+)-$\alpha$-Pinenoxid = 95 % der Theorie erhalten.

Beispiel 2

In der unter Beispiel 1 beschriebenen Apparatur werden 1,1 Mol = 91,3 g Cyclohexen, 99 %ig, 228,3 g Chloroform und 137,0 g Wasser vorgelegt. Anschließend gibt man innerhalb von 30 Minuten 1,0 Mol = 190,1 g Gleichgewichtsperessigsäure, 40 %ig, hinzu, während gleichzeitig der pH-Wert der Reaktionslösung durch Zugabe von ca. 95 g 50 %iger Natronlauge bei pH = 5,0 gehalten wird. Die Reaktionstemperatur wird während dieser Zeit durch Kühlung auf 20 °C gehalten.

Man läßt noch 2 Stunden bei 40 °C unter Rühren reagieren und trennt die Phasen. Die organische Phase wird dreimal mit je 80 g Wasser gewaschen, wobei bei der zweiten Wäsche mittels Natronlauge ein pH-Wert von 7,0 eingestellt wird.

Die gewaschene organische Phase wird destillativ aufgearbeitet. Hierbei werden bei einem Olefin-Umsatz von 82 % vom Einsatz 85,9 g = 0,875 Mol Cyclohexenoxid = 97 % der Theorie erhalten.

Beispiel 3

In der unter Beispiel 1 beschriebenen Apparatur werden 0,5 Mol = 111,3 g(3-Cyclohexenylmethyl-3-Cyclohexenylcarboxylat (THB)$_2$ -Ester), 99 %ig, 275,4 g Chloroform und 165,2 g Wasser vorgelegt. Anschließend gibt man innerhalb von 30 Minuten 1,1 Mol = 209,1 g Gleichgewichtsperessigsäure, 40 %ig, hinzu, während gleichzeitig der pH-Wert der Reaktionslösung durch Zugabe von ca. 21 g 50 %iger Natronlauge bei pH = 4,0 gehalten wird. Die Reaktionstemperatur wird während dieser Zeit durch Kühlung auf 20 °C gehalten.

Man läßt noch 2 Stunden bei 50 °C unter Rühren reagieren und trennt die Phasen. Die organische Phase wird dreimal mit je 80 g Wasser gewaschen, wobei bei der zweiten Wäsche mittels Natronlauge ein pH-Wert von 7,0 eingestellt wird.

Die gwaschene organische Phase wird destillativ aufgearbeitet. Hierbei werden bei einem 100 %igen Olefin-Umsatz erhalten:

5,3 g = 0,02 Mol = 4,5 % d.Theorie (THB)$_2$ -Ester-monoepoxid
105,0 g = 0,42 Mol = 83 % d.Theorie (THB)$_2$ -Ester-diepoxid =
3,4-Epoxycyclohexylmethyl-3,4-Epoxycyclohexancarboxylat.

Beispiel 4

In der unter Beispiel 1 beschriebenen Apparatur werden 1,0 Mol = 79,3 g 2-Methyl-2-buten, 88,4 %ig, 315,6 g Methylenchlorid und 105,2 g Wasser vorgelegt. Anschließend gibt man innerhalb von 30 Minuten 1,0 Mol = 196,0 g Gleichgewichtsperessigsäure, 38,8 %ig, hinzu, während gleichzeitig der pH-Wert der Reaktionslösung durch Zugabe von ca. 107 g 50 %iger Natronlauge bei pH = 5,5 gehalten wird. Die Reaktionstemperatur wird während dieser Zeit durch Kühlung auf 20 °C gehalten.

Man läßt noch 1 Stunde bei 20 °C unter Rühren reagieren und trennt die Phasen. Die organische Phase wird dreimal mit je 80 g Wasser gewaschen, wobei bei der zweiten Wäsche mittels Natronlauge ein pH-Wert von 7,0 eingestellt wird.

Die gewaschene organische Phase wird destillativ aufgearbeitet. Hierbei werden bei einem Olefin-Umsatz von 87 % vom Einsatz 73,4 g = 0,853 Mol Isoamylenoxid = 98 % der Theorie erhalten.

**Patentansprüche**

1. Verfahren zur Herstellung von Epoxiden durch Umsetzung von Olefinen mit Percarbonsäuren in Gegenwart säurebindender Mittel,
   dadurch gekennzeichnet,
   daß man die Epoxidationsreaktion in einem feststofffreien oder nahezu von Feststoffen freien wäßrig-organischen Zweiphasensystem bei einem konstanten pH-Wert von 3 bis 6 durchführt, wobei das Verhältnis von eingesetztem Olefin zu dem Gesamtwasser aus zugefügtem Wasser, Wasseranteil der Peressigsäure und der wäßrigen Lösung des Säurebinders auf 1 zu 0,5 bis 1 zu 5 einstellt und der vom jeweiligen Olefin abhängige, in einem Vorversuch festgestellte optimale pH-Wert während der gesamten Reaktionsdauer durch Zugabe von alkalisch wirkenden Stoffen gehalten wird.

2. Verfahren nach Anspruch 1,
   dadurch gekennzeichnet,
   daß man die Epoxidierung mit sogenannter Gleichgewichtsperessigsäure durchführt, die neben Peressigsäure noch Essigsäure, Wasser, Wasserstoffperoxid und Mineralsäure enthält.

3. Verfahren nach Anspruch 1 und 2,
   dadurch gekennzeichnet,
   daß man den pH-Wert durch Zugabe von wäßrigem Alkalihydroxid, bevorzugt Natriumhydroxid, einstellt und aufrechterhält.

4. Verfahren nach Anspruch 1 bis 3,
   dadurch gekennzeichnet,
   daß man bei Anwesenheit eines organischen Lösungsmittels ein Verhältnis von Olefin zu Lösungsmittel von 1 zu 0,5 bis 1 zu 10 verwendet.

5. Verfahren nach Anspruch 1 bis 4,
   dadurch gekennzeichnet,
   daß man das Verhältnis von Olefindoppelbindung zu Gleichgewichtsperessigsäure auf 1 zu 1 einstellt.

**Claims**

1. A process for the preparation of epoxides by the reaction of olefins with percarboxylic acids in the presence of acid-binding agents, characterised in that the epoxidation reaction is carried out in an aqueous organic diphasic system which is free from solid content or almost free from solid content at a constant pH of from 3 to 6, the ratio of olefin put into the process to the total quantity of water composed of added water and water content of the peracetic acid and of the aqueous solution of the acid binder being adjusted to a value from 1 : 0.5 to 1 : 5 and the optimum pH, which depends on the olefin used and is determined in a preliminary test, being maintained during the whole reaction time by the addition of substances which are alkaline in their action.

2. A process according to Claim 1, characterised in that epoxidation is carried out with so-called equilibrium peracetic acid containing acetic acid, water, hydrogen peroxide and mineral acid in addition to peracetic acid.

3. A process according to Claims 1 and 2, characterised in that the pH is adjusted and maintained by the addition of aqueous alkali metal hydroxide, preferably sodium hydroxide.

4. A process according to Claims 1 to 3, characterised in that a ratio of olefin to solvent of from 1 : 0.5 to 1 : 10 is employed in the presence of an organic solvent.

5. A process according to Claims 1 to 4, characterised in that the ratio of olefin double bonds to equilibrium per acetic acid is adjusted to 1 : 1.

**Revendications**

1. Procédé de production d'époxydes par transformation d'oléfines par des acides percarboxyliques en présence d'un agent liant acide, caractérisé en ce qu'on réalise la réaction d'époxydation dans un système à deux phases, organique aqueux, exempt de matières solides ou presque sans matière solide, avec une valeur de pH constante entre 3 et 6, dans lequel le rapport entre l'oléfine mise en jeu et l'eau totale provenant de l'eau ajoutée, de la quote part d'eau de l'acide peracétique et de la solution aqueuse du liant acide, est ajusté à une valeur de 1 à 0,5 jusqu'à 1 à 5 et dans lequel la valeur de pH optimale fonction de l'oléfine correspondante, déterminée lors d'un essai préliminaire, est maintenue pendant toute la durée de la réaction en ajoutant des matières à action alcaline.

2. Procédé selon la revendication 1, caractérisé en ce qu'on réalise l'époxydation avec de l'acide appelé acide peracétique d'équilibre, qui contient en plus de l'acide peracétique, de l'acide acétique, de l'eau, du peroxyde d'hydrogène et de l'acide minéral.

3. Procédé selon les revendications 1 et 2, caractérisé en ce qu'on ajoute la valeur de pH par addition d'hydroxyde alcalin aqueux, de préférence de l'hydroxyde de sodium et on le maintient.

4. Procédé selon les revendications 1 à 3, caractérisé en ce qu'on utilise, en présence d'un solvant organique, un rapport entre oléfine et solvant de 1 à 0,5 jusqu'à 1 à 10.

5. Procédé selon les revendications 1 à 4, caractérisé en ce qu'on règle le rapport de liaison double d'oléfine à acide peracétique d'équilibre à 1 : 1.